# FASCICULE DE BREVET EUROPEEN

(11) **EP 1 150 732 B1**
(45) Date de publication et mention de la délivrance du brevet: **25.10.2006**
(21) Numéro de dépôt: 00905107.9
(22) Date de dépôt: 10.02.2000
(51) Int. Cl.: A61M 16/00

(54) **DISPOSITIF D'ALIMENTATION EN GAZ POUR APNEES DU SOMMEIL**
VORRICHTUNG ZUR GASZUFUHR BEI SCHLAFAPNOE
GAS SUPPLY DEVICE FOR SLEEP APNEA

(30) Priorité: 12.02.1999 FR 9906516
(43) Date de publication de la demande: 07.11.2001
(73) Titulaire: Mallinckrodt Developpement France, 54600 Villers-les-Nancy (FR)
(72) Inventeur: NADJAFIZADEH, Hossein, F-54600 Villers-les-Nancy (FR); NICOLAZZI, Pascal, F-54520 Laxou (FR); GRILLER LANOIR, Véronique, F-25000 Besançon (FR)
(74) Mandataire: Geismar, Thierry
(86) Numéro de dépôt international: PCT/FR2000/000333
(87) Numéro de publication internationale: WO 2000/047261

(56) Documents cités:
- WO-A-92/11054
- WO-A-92/22244
- WO-A-94/06499
- WO-A-94/23780
- WO-A-97/28838
- US-A- 5 823 187

## Description

L'invention concerne un procédé de commande d'un appareil de fourniture de pression d'air à un patient souffrant de troubles du sommeil.

L'invention concerne également un appareil de fourniture de pression d'air à un patient souffrant de troubles du sommeil.

Ces troubles du sommeil sont respiratoires et tendent à réveiller intempestivement le patient.

Ce sont par exemple les apnées, les hypopnées, les vibrations acoustiques ou ronflements, la limitation du flux respiratoire dues à un resserrement des voies aériennes supérieures du patient.

Le document US-A-5 458 137 décrit un procédé et un dispositif pour contrôler la respiration en cas de troubles du sommeil, qui utilisent des niveaux de pression multiples et variables. WO 97/28838 divulgue les caractéristiques du préambule de la revendication 1.

Une source de pression fournit un gaz respirable comprimé à une pression relativement basse aux voies aériennes de l'utilisateur.

Des capteurs de pression surveillent les pressions et les convertissent en signaux électriques.

Les signaux électriques sont filtrés et traités pour extraire des caractéristiques spécifiques telles que la durée et des niveaux d'énergie.

Si ces caractéristiques dépassent des seuils choisis de durée et de niveau d'énergie au-delà d'une période minimum de temps, le microprocesseur indique la présence d'un trouble respiratoire du sommeil.

Si un nombre choisi de ces événements apparaît pendant une période de temps choisie, le microprocesseur ajuste la pression fournie par la source.

Le document US-A-5 490 502 décrit un procédé et un appareil pour optimiser la pression positive commandée afin de minimiser le débit d'air provenant d'un générateur tout en assurant que la limitation de débit dans les voies aériennes du patient ne se produit pas.

Il y est prévu de détecter la limitation de débit en analysant une onde de débit respiratoire.

Dès que la présence d'une limitation de débit a été analysée, le système détermine une action à effectuer pour l'ajustement de la pression positive commandée.

La pression est augmentée, diminuée ou maintenue selon que la limitation de débit a été détectée et en fonction des actions précédentes mises en oeuvre par le système.

Citons également les documents US-A-5 335 654, EP-A-661 071 et EP-A-651 971.

L'invention vise à proposer un appareil de fourniture de pression d'air permettant la détection des apnées centrales.

L'invention concerne un appareil de fourniture de pression d'air à un patient souffrant de troubles du sommeil tels qu'apnée centrale selon la revendication 1.

Le patient porte un masque par lequel de l'air sous pression est fourni à ses voies aériennes supérieures par l'appareil.

Selon l'invention, il est prévu un algorithme de commande utilisant un signal de débit de sortie de l'appareil pour la détection d'apnée, d'hypopnée, d'événements de limitation de débit, de fuites, et utilisant l'analyse d'une information de pression pour déterminer la présence d'un ronflement, également appelé vibrations acoustiques.

La pression fournie aux voies aériennes supérieures du patient par l'appareil peut être maintenue constante, être augmentée ou diminuée en fonction de la détermination de l'événement qui a été effectuée par l'algorithme de commande.

Ainsi, si aucune respiration n'est détectée par l'algorithme de commande en un temps minimum prédéterminé dépendant d'un temps de respiration moyen calculé, on détermine la présence d'une apnée.

Ce temps minimum prédéterminé de détection d'apnée est par exemple égal à une constante de temps, par exemple 10 s, ajoutée à un facteur de proportionnalité multiplié par le temps de respiration moyen calculé, ce facteur étant par exemple égal à 5/8.

Pour chaque apnée, le signal de débit de sortie est amplifié et filtré pour déterminer la présence ou l'absence d'oscillations cardiaques.

Si des oscillations cardiaques ont été détectées pendant le dernier intervalle de temps écoulé, par exemple égal à 5 s, alors l'apnée est classée comme étant centrale et aucune commande ne se produit dans l'algorithme.

Si aucune oscillation cardiaque n'a été détectée dans cet intervalle de temps, l'apnée est classée comme étant obstructive, et la pression est augmentée d'une valeur prédéterminée une première fois et, durant la même apnée, deux autres fois régulièrement, par exemple toutes les 15 s.

L'algorithme de commande compare des variations de débit crête à crête durant la dernière respiration du patient par rapport à un nombre prédéterminé de respirations précédentes, par exemple égal à 8.

Après chaque respiration, on effectue une classification en :
- respiration normale, si la dernière valeur de débit crête à crête est comprise dans une fourchette déterminée par rapport à la valeur moyenne sur les 8 respirations précédentes, par exemple de 40 % à 150 % ou 140 % de celles-ci ;
- respiration hypopnéique, si la dernière valeur de débit est en-deçà de cette plage;
- respiration hyperpnéique, si la dernière valeur de débit est au-delà de cette plage.

Une détermination d'hypopnée est effectuée si la détection de respiration hypopnéique s'est produite pendant au moins un temps déterminé, par exemple 10 s, et prend fin après un nombre déterminé de respirations normales ou hyperpnéiques, par exemple égal à 2.

Une détermination d'hypopnée conduit à une augmentation de pression déterminée, par exemple de 1 cm H2O d'abord, puis, durant la même hypopnée, à une augmentation de pression d'une autre valeur déterminée et ce régulièrement, par exemple de 0,5 cm H2O toutes les deux respirations hypopnéiques.

L'algorithme de commande analyse et compare, respiration par respiration, la forme d'onde du débit respiratoire avec une forme d'onde sinusoïdale de même période et de même pente.

Après la comparaison basée sur deux critères de forme de débit, chaque respiration est d'abord classée en tant que normale, intermédiaire ou à débit limité.

Une classification finale basée sur la combinaison de la classification de flux et de l'occurrence de ronflements, change le classement des respirations de normales en intermédiaires, respectivement d'intermédiaires en respiration à débit limité.

Il est décidé d'un traitement lorsqu'un certain nombre, par exemple 2, respirations successives à débit limité ou un certain nombre, par exemple 5, de respirations successives intermédiaires ont lieu après par exemple deux respirations normales.

Ce traitement provoque une augmentation de pression déterminée, répétée régulièrement un certain nombre de fois, par exemple de 0,3 cm H2O trois fois toutes les deux respirations.

Pour chaque respiration, le signal de pression est amplifié et filtré pour détecter la présence ou l'absence de vibrations acoustiques ou ronflement.

Une détermination d'un ronflement valide est effectuée par l'algorithme de commande, si la vibration acoustique détectée s'est produite au moins pendant un certain temps, par exemple 7 % de la durée moyenne des trois dernières respirations, et avec une période inférieure à un facteur proportionnel à ce temps moyen, par exemple 120 % de celui-ci.

Dans le cas d'un ronflement valide, l'algorithme augmente la pression d'une valeur déterminée, par exemple de 1 cm H2O, si la dernière commande due à un ronflement s'est produite depuis plus d'un temps déterminé, par exemple 1 minute.

On détermine une fuite moyenne comme étant égale au débit moyen durant la respiration.

L'algorithme de commande compare continûment la fuite actuelle à une limite de fuite, laquelle limite peut être réglée à partir de la pression.

Si la fuite actuelle dépasse la limite, on bloque toutes les commandes d'augmentation de pression générées à la suite de détections d'événements.

Après une détection d'une apnée ou d'un événement de ronflement ou une commande en hypopnée ou une décision de traitement, l'algorithme diminuera la pression d'une valeur déterminée, par exemple de 0,5 cm H2O, dans une première étape après un temps déterminé, par exemple 5 minutes, et régulièrement pour les diminutions suivantes, par exemple toutes les minutes.

Une pression de maintien déterminée, par exemple de 8 cm H2O est fournie par l'appareil si aucune respiration n'a été détectée pendant un temps déterminé, par exemple de deux minutes, ou si la pression fournie a été supérieure ou égale à une valeur déterminée pendant un temps déterminé, par exemple à 17 cm H2O pendant 10 ou 30 minutes.

Un avantage du procédé est une adaptation automatique des critères de détection aux caractéristiques respiratoires du patient.

Ainsi, toute modification du rythme respiratoire est prise en compte par l'algorithme pour effectuer la détection.

Le fait de faire intervenir une valeur moyenne de temps de cycle respiratoire sur un certain nombre de cycles respiratoires précédents a comme effet le suivi régulier des variations du cycle et d'amplitude respiratoire et une meilleure détection.

L'invention sera mieux comprise à la lecture de la description qui va suivre, faite en référence aux figures.

La figure 1 est un schéma montrant l'appareil de fourniture de pression d'air au patient.

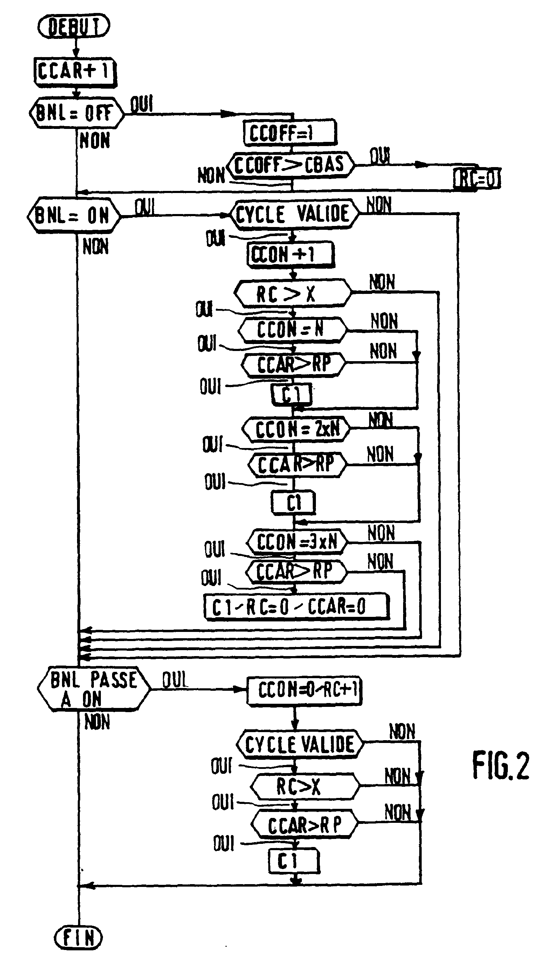

La figure 2 représente un algorithme de prise de décision en vue d'une première commande d'augmentation de pression.

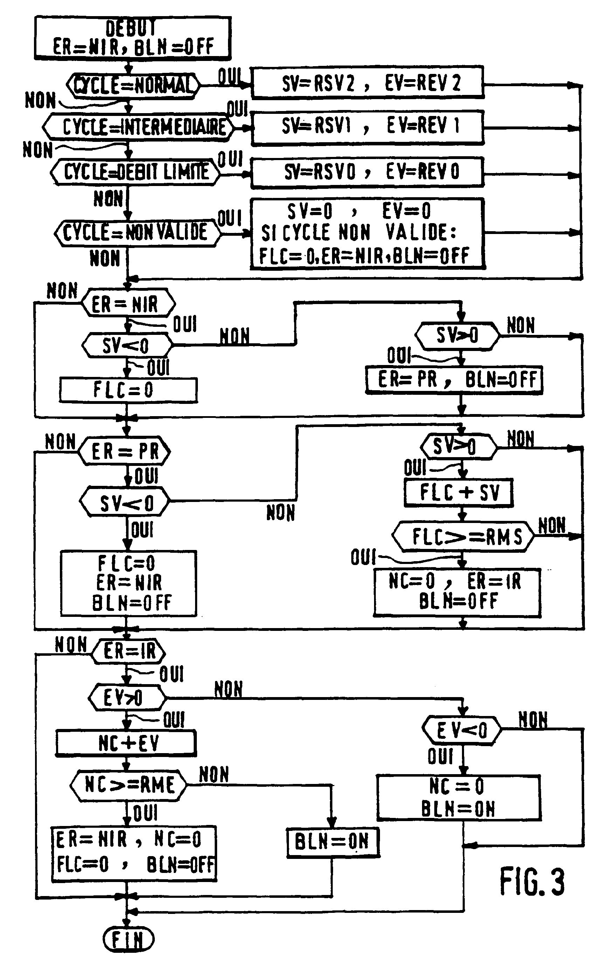

La figure 3 représente un algorithme d'indication d'apparitions de troubles.

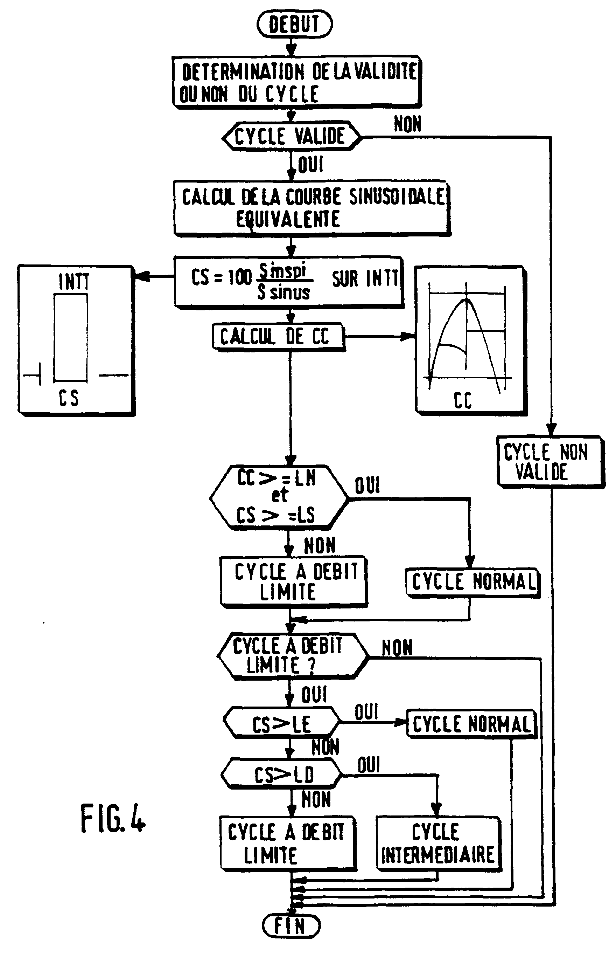

La figure 4 représente un algorithme de qualification de respiration.

La figure 5 représente un algorithme de détection d'apnée centrale et obstructive et de commande de pression en fonction du résultat de ces détections, ainsi qu'un algorithme de diminution de pression selon l'apparition précédente ou non d'évènements représentatifs de troubles du sommeil.

La figure 6 représente un algorithme de qualification de cycle de ventilation normale, à hyperventilation ou à hypoventilation.

La figure 7 représente un algorithme de détection de respiration hypopnéique.

La figure 8 représente un algorithme de détection de respiration hyperpnéique.

La figure 9 représente un algorithme de détection de respiration normale.

La figure 10 représente un algorithme de détection de pression élevée.

La figure 11 représente un algorithme de détection de fuite du masque.

La figure 12 représente un algorithme de détection de vibrations acoustiques.

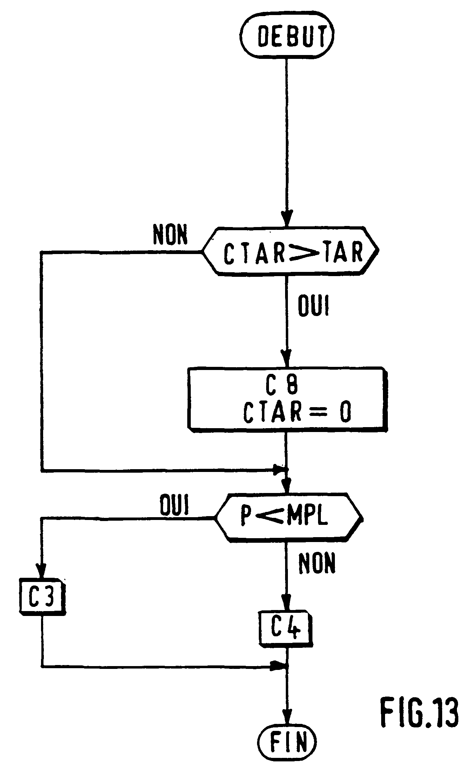

La figure 13 représente un algorithme de diminution de pression en cas de détection de vibrations acoustiques.

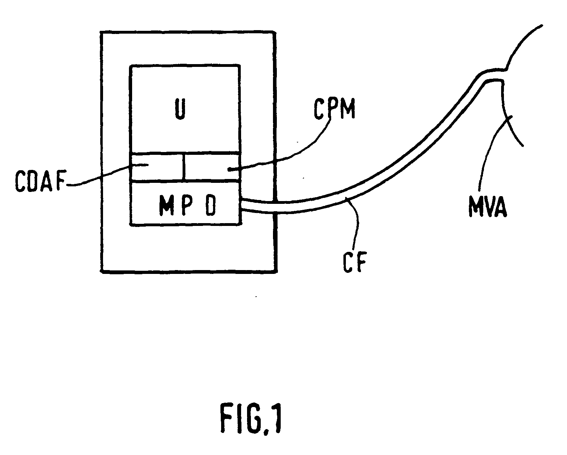

A la figure 1, l'appareil de fourniture de pression d'air à un patient comporte une unité U centrale de traitement et de commande de pression, un module MPD commandé de fourniture de pression, un masque MVA pour les voies aériennes supérieures du patient, un conduit CF de fourniture de pression d'air du module MPD au masque MVA.

On mesure le débit d'air fourni au patient et la pression d'air régnant dans le masque MVA, par un capteur CDAF de débit d'air fourni, relié à l'unité centrale U et par un capteur CPM de pression dans le masque MVA, relié à l'unité centrale U.

On détermine à partir des variables mesurées, si des événements représentatifs de troubles du sommeil apparaissent ou non.

Les algorithmes du procédé suivant l'invention sont mis en oeuvre par un logiciel intégré à l'unité centrale U.

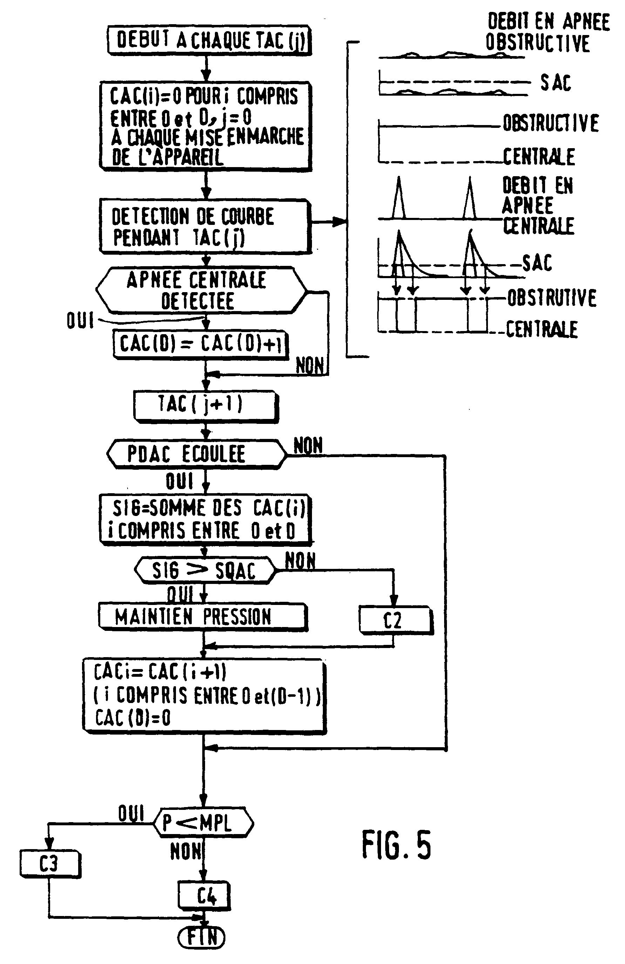

A la figure 5, on détecte les apnéées obstructives et les apnées centrales.

L'algorithme représenté à la figure 5 est effectué pendant chacun de plusieurs (NINT) intervalles de temps TAC(j) consécutifs prédéterminés.

Les intervalles de temps TAC(j) consécutifs prédéterminés sont ceux compris dans une période prédéterminée PDAC de détection d'apnée.

Dans cet algorithme, on détecte par exemple par des moyens matériels tels que filtres analogiques ou numériques les oscillations de la courbe de débit mesurée, qui sont de fréquences comprises dans une plage P2 de fréquence.

Puis on détecte si l'amplitude des oscillations détectées de la courbe de débit mesurée passe successivement au-dessus puis au-dessous d'un premier seuil SAC prédéterminé d'apnée centrale ou si cette amplitude reste inférieure au premier seuil SAC d'apnée centrale, comme représenté schématiquement à droite de la figure 5 par :
- l'allure d'une courbe de débit en apnée obstructive (courbe constamment en deçà du premier seuil SAC) ;
- l'allure d'une courbe de débit en apnée centrale (courbe passant plusieurs fois successivement au-dessus puis au-dessous du premier seuil SAC).

En présence d'au moins une détection d'un passage au-dessus puis au-dessous du premier seuil SAC, on compte une détection CAC(D) d'apnée centrale.

Puis, à chaque période PDAC de détection d'apnée.
- on fait la somme SIG des nombres CAC(i) de détections d'apnée centrale comptées, successivement sur les (D+1) dernières périodes de détection d'apnée,
- on commande C2 une deuxième augmentation prédéterminée de pression d'air délivré si la somme SIG des nombres CAC(i) de détections comptées est inférieure ou égaie à un deuxième seuil SQAC prédéterminé de qualification d'apnée centrale ;
- on commande un maintien de pression d'air délivré, si la somme SIG des nombres CAC(i) de détections comptées est supérieure au deuxième seuil SQAC.

Dans une réalisation, le deuxième seuil SQAC de qualification d'apnée centrale est compris entre 0 et 50, et est par exemple sensiblement égal à 10.

Les intervalles de temps TAC(j) consécutifs prédéterminés correspondent à dix (NINT) intervalles de temps consécutifs de chacun sensiblement 100 ms, la période PDAC de détection d'apnée correspondant sensiblement à 1 s.

La deuxième commande C2 d'augmentation de pression est comprise entre 1 et 10 mbar et est par exemple sensiblement égale à + 1 mbar.

Le nombre (D+1) de périodes PDAC de détection d'apnée, sur lesquelles on fait la somme des nombres CAC(i) comptés de détection d'apnée centrale est sensiblement égal à 5.

La deuxième plage P2 de fréquence d'oscillations est comprise entre sensiblement 2,5 et 47 Hz.

Les nombres CAC(i) comptés de détections d'apnée centrale sont remis à 0 lors de la mise en marche de l'appareil.

Il est également représenté à la figure 5 un algorithme de diminution de pression selon l'apparition précédente ou non d'évènements représentatifs de troubles du sommeil.

Selon cet algorithme, représenté au bas de la figure 5, on compare la pression P mesurée à une valeur MPL prédéterminée de pression.

Après la détermination de l'apparition de l'un ou plusieurs des événements,
- si la pression P mesurée est inférieure à la valeur MPL prédéterminée, on effectue une troisième commande C3 de diminution prédéterminée de pression,
- si la pression P mesurée est supérieure ou égale à la valeur MPL prédéterminée, on effectue une quatrième commande C4 prédéterminée de diminution de pression,
- puis, si aucune apparition d'événement n'a été détectée après une ou plusieurs des commandes C3 ; C4 de diminution de pression, on effectue la quatrième commande C4 prédéterminée de diminution de pression.

La quatrième commande C4 de diminution de pression est telle qu'elle provoque une diminution de pression plus grande par unité de temps que celle provoquée par la troisième commande C3.

Dans une réalisation, la quatrième commande C4 de diminution de pression est sensiblement de -0,5 mbar / 1 minute et la troisième commande C3 de diminution de pression est sensiblement de - 0,5 mbar / 5 minutes, la valeur MPL comparative de pression est comprise entre 4 et 19 mbar et est par exemple sensiblement égale à 17 mbar.

Cet algorithme de diminution de pression en fonction de l'apparition ou non d'évènements est mis en oeuvre après celui de détection d'apnées centrales et obstructives comme représenté à la figure 5 mais est également mis en oeuvre, dans des réalisations non représentées, après les autres algorithmes tels que :
- celui de prise de décision de traitement, lorsque l'indicateur BLN est passé du premier état ON au deuxième état OFF ;
- celui de détection de respiration hypopnéique décrit ci-après ;
- celui de détection de vibrations acoustiques décrit ci-après.

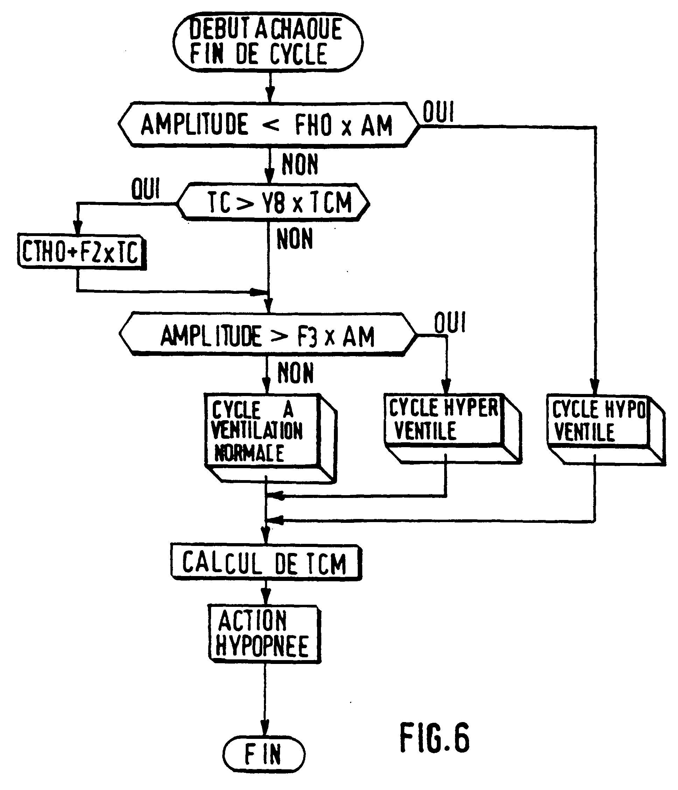

Aux figures 6 à 9, on qualifie les cycles respiratoires d'hyperventilés, d'hypoventilés ou de cycles à ventilation normale et on génère des commandes de presssion en fonction des qualifications effectuées.

On calcule à chaque fin de cycle respiratoire mesuré, l'amplitude moyenne AM sur un quatrième nombre prédéterminé Y4 de cycles respiratoires précédents.

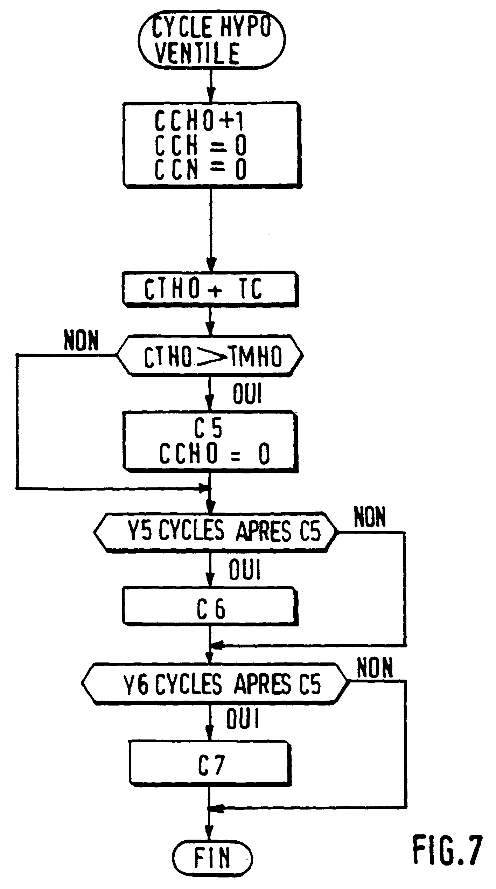

Comme représenté à la figure 7, si l'amplitude mesurée du dernier cycle respiratoire est inférieure à l'amplitude moyenne AM calculée multipliée par un premier facteur d'hypopnée prédéterminé FHO, alors on ajoute à un compteur CTHO de temps en hypopnée la durée TC du dernier cycle respiratoire mesuré,
- si la valeur actuelle du compteur de temps en hypopnée CTHO est supérieure ou égale à un temps minimum d'hypopnée TMHO, on commande par une commande C5 une cinquième augmentation prédéterminée de pression.
- après la fin d'un cinquième nombre prédéterminé Y5 de cycles respiratoires suivant la cinquième commande C5 d'augmentation de pression, on commande C6 une sixième augmentation prédéterminée de pression ;
- après la fin d'un sixième nombre prédéterminé Y6 de cycles respiratoires, supérieur au cinquième nombre Y5, suivant la cinquième commande C5 d'augmentation de pression, on commande par une commande C7 une septième augmentation de pression.

Le compteur de temps en hypopnée CTHO étant initialisé à 0 lors de la mise en marche de l'appareil.

Dans une réalisation, le quatrième nombre déterminé Y4 de cycles respiratoires de calcul d'amplitude moyenne est sensiblement égal à 8.

Le premier facteur prédéterminé FHO d'hypopnée est compris entre 1 et 100 % et est par exemple sensiblement égal à 40 %.

Le temps minimum d'hypopnée TMHO est compris entre 1 s et 25 s et est par exemple sensiblement égal à 10 s.

Les cinquième et sixième nombres prédéterminés Y5 ; Y6 de cycles respiratoires sont sensiblement égaux à respectivement 2 et 4.

La cinquième augmentation C5 prédéterminée de pression est comprise entre 0,1 mbar et 10 mbar et est par exemple sensiblement égale à + 1 mbar.

Les sixième et septième augmentations C6 ; C7 prédéterminées de pression sont chacune inférieures à la cinquième commande C5 et sont par exemple chacune sensiblement égales à la moitié de la cinquième augmentation C5 de pression.

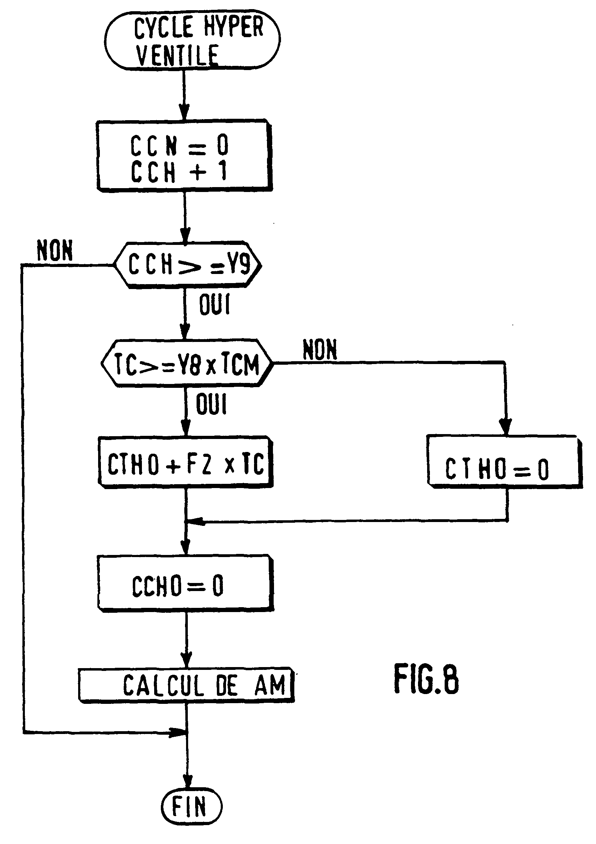

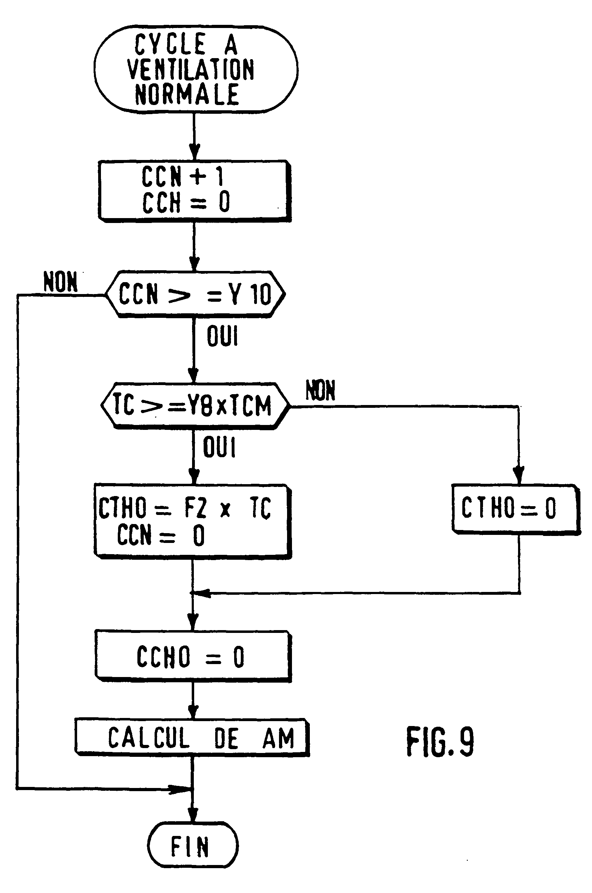

Comme représenté aux figures 8 et 9, si l'amplitude mesurée du dernier cycle respiratoire est supérieure ou égale à l'amplitude moyenne AM calculée multipliée par la premier facteur d'hypopnée FHO, alors on calcule le temps TCM de cycles respiratoires moyen sur un septième nombre prédéterminé Y7 de cycles précédents.

Si la durée mesurée TC du dernier cycle est supérieure à un huitième nombre prédéterminé Y8 multiplié par le temps de cycle respiratoire moyen calculé TCM, on ajoute au compteur de temps en hypopnée CTHO la durée mesurée TC du dernier cycle, multipliée par un deuxième facteur F2 d'hypopnée.

Si l'amplitude mesurée du dernier cycle respiratoire mesuré est supérieure à un troisième facteur F3 d'hyperventilation, supérieur au premier facteur FHO d'hypopnée, multiplié par l'amplitude moyenne calculée AM. on qualifie le dernier cycle d'hyperventilé, on incrémente d'une unité un compteur de cycles hyperventilés CCH, on remet à 0 un compteur CCN de cycles à ventilation normale et
■ si la valeur du compteur CCH de cycles hyperventilés est supérieure ou égale à un neuvième nombre prédéterminé Y9,
   ■ si la durée du dernier cycle TC est supérieure ou égale au huitième nombre Y8 multiplié par le temps de cycle moyen calculé TCM, on ajoute au compteur CTHO de temps en hypopnée le deuxième facteur F2 multiplié par la durée du dernier cycle respiratoire TC,
   ■ et sinon, on remet à 0 le compteur CTHO de temps en hypopnée ;
puis on remet à 0 un compteur CCHO de cycles hypoventilés et on calcule l'amplitude moyenne AM de cycle respiratoire sur le nombre prédéterminé Y4 de cycles respiratoires précédents.

Si l'amplitude mesurée du dernier cycle respiratoire mesuré est inférieure ou égale au troisième facteur F3 multiplié par l'amplitude moyenne calculée AM, on qualifie le dernier cycle de cycle à ventilation normale, on remet à 0 le compteur CCH de cycles hyperventilés et on incrémente d'une unité le compteur CCN de cycles à ventilation normale, et
■ si la valeur du compteur CCN de cycles à ventilation normale est supérieure ou égale à un dixième nombre Y10 prédéterminé,
   ■ si la durée du dernier cycle TC est supérieure ou égale au huitième nombre Y8 multiplié par le temps de cycle moyen calculé TCM, on affecte au compteur CTHO de temps en hypopnée le deuxième facteur F2 multiplié par la durée du dernier cycle TC et on remet à 0 le compteur CCN de cycle à ventilation normale,
   ■ et sinon, on remet à 0 le compteur CTHO de temps en hypopnée ;
puis on remet à 0 le compteur CCHO de cycles hypoventilés et on calcule l'amplitude moyenne du cycle respiratoire sur le nombre prédéterminé Y4 de cycles respiratoires.

Dans une réalisation, le deuxième facteur F2 est sensiblement égal à 5/8.

Le troisième facteur d'hyperventilation F3 est compris entre 100 % et 200 % et est par exemple sensiblement égal à 140 %.

Les septième, huitième, neuvième et dixième nombres prédéterminés Y7; Y8 ; Y9 ; Y10 sont respectivement sensiblement égaux à 3 ; 2 ; 2 ; et 2.

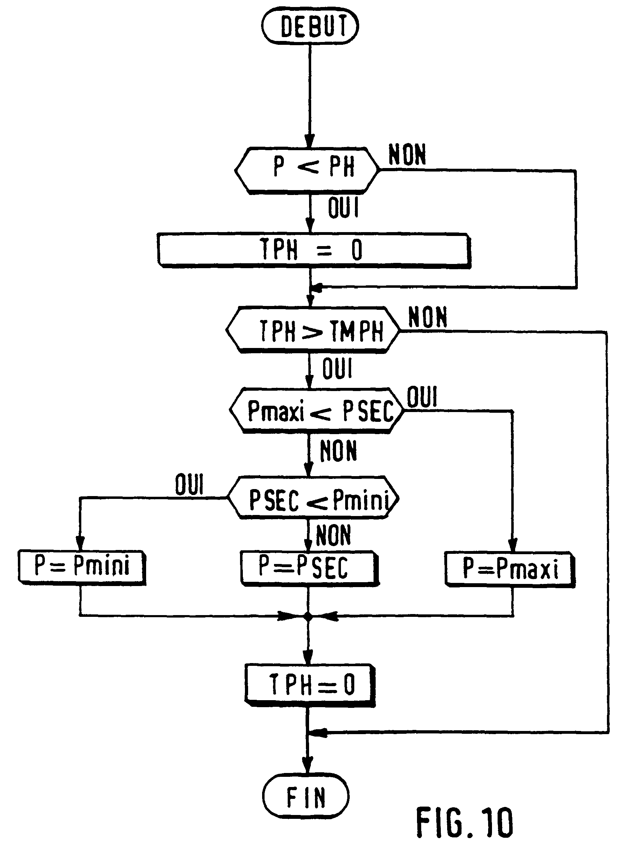

A la figure 10, on détecte si la pression est trop élevée.

Si la pression mesurée P est inférieure à une valeur de pression haute PH prédéterminée, on remet à 0 un compteur de temps en pression haute TPH.

Si la valeur du compteur de temps en pression haute TPH est supérieure à un temps maximum de pression haute TMPH et
- si la valeur maximale de pression réglée Pmaxi est inférieure à une valeur de pression de sécurité PSEC prédéterminée, on commande la pression P à cette valeur maximale de pression réglée Pmaxi;
- si la valeur minimale de pression réglée Pmini est supérieure à une valeur de pression de sécurité PSEC prédéterminée, on commande la pression P à cette valeur minimale de pression réglée Pmini;
- si les deux précédentes conditions ne sont pas réalisées, on commande la pression P à la valeur de pression de sécurité PSEC,
puis
- on remet à 0 le compteur de temps en pression haute TPH.

Dans une réalisation, la valeur de pression haute PH est comprise entre 10 mbar et 25 mbar et est par exemple sensiblement égale à 17 mbar.

Le temps maximum de pression haute TMPH est compris entre 1 et 100 minutes et est par exemple sensiblement égal à 10 minutes ou 30 minutes.

La valeur de pression de sécurité PSEC est sensiblement égale à 8 mbar.

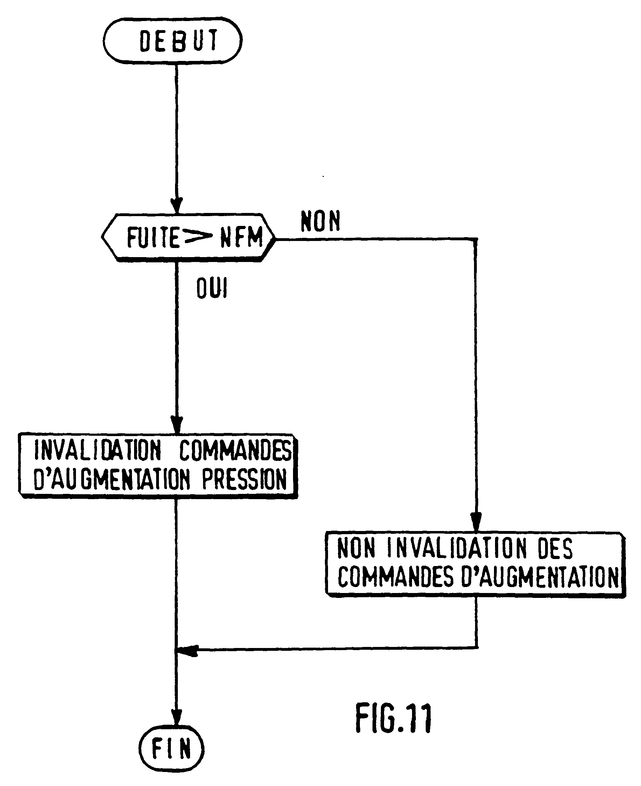

A la figure 11, on mesure une fuite d'air, sensiblement égale au débit moyen pendant la respiration du patient.

Si la fuite mesurée d'air est supérieure à un niveau prédéterminé de fuite NFM, on invalide les commandes d'augmentation de pression.

Dans une réalisation, NFM = A x Pfiltrée + B.

Selon cette formule, le niveau prédéterminé de fuite NFM est sensiblement égal à un coefficient A de fuite multiplié par une pression d'air filtrée dans le masque, ajouté à un coefficient B additif de fuite, le coefficient A de fuite étant compris entre 0 et 10 I/minute.mbar et étant par exemple sensiblement égal à 2,5 l/minute.mbar.

Le coefficient B additif de fuite est compris entre 0 et 100 l/mn et est par exemple sensiblement égal à 50 l/mn.

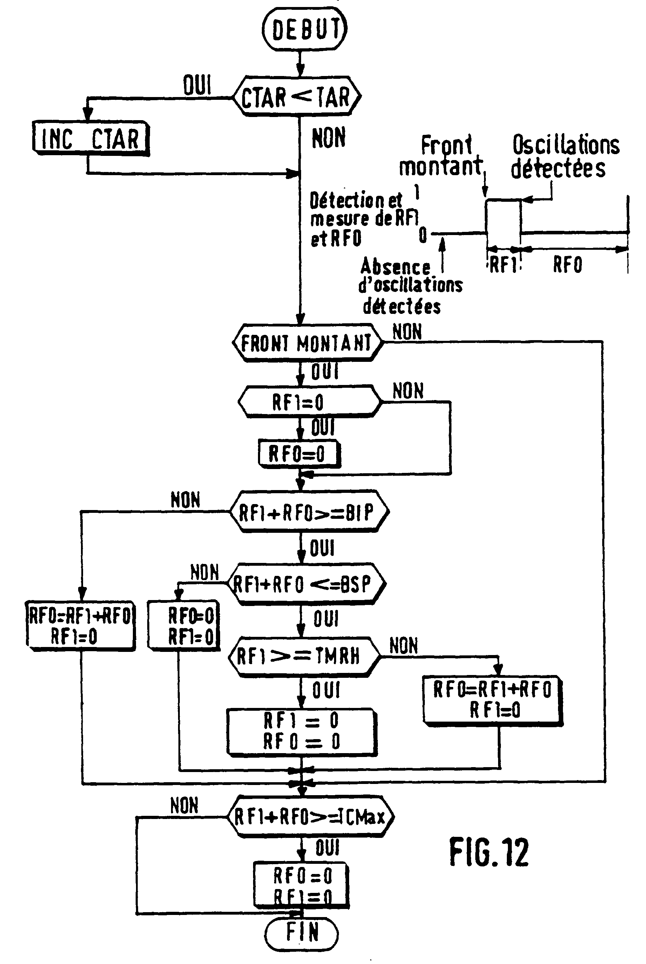

A la figure 12, on détecte si la courbe de pression mesurée présente des oscillations, telles que de vibrations acoustiques, comprises dans une plage P1 de fréquence.

Cette détection est effectuée par exemple par des moyens matériels tels que filtres analogiques ou numériques.

On mesure le temps RF1 de présence d'oscillations détectées entre deux absences successives d'oscillations détectées et le temps RF0 d'absence d'oscillations détectées entre deux présences successives d'oscillations détectées ;

Si la somme des temps mesurés d'absence et de présence d'oscillations détectées RF0 ; RF1 est comprise dans une plage temporelle prescrite BIP ; BSP.

Si le temps de présence d'oscillations RF1 mesuré est supérieur ou égal à un temps minimum d'oscillations TMRH et si la valeur d'un compteur CTAR de temps écoulé depuis l'avant-dernière fois que les conditions temporelles précédentes ont été réalisées, est supérieure à un temps d'attente prescrit TAR, on commande C8 une huitième augmentation prédéterminée de pression et on remet le compteur de temps écoulé CTAR à 0.

Les algorithmes de détection de vibrations acoustiques et de commande en cas de vibrations acoustiques sont mis en oeuvre à intervalles de temps prescrits, notamment régulièrement et par exemple toutes les 100ms.

Au début de l'algorithme de détection de vibrations acoustiques représenté à la figure 12, si la valeur du compteur CTAR de temps écoulé est inférieure au temps d'attente prescrit TAR, on incrémente (INC CTAR) ce compteur de l'intervalle de temps prescrit mentionné ci-dessus.

Si la somme des temps mesurés de présence et d'absence d'oscillations détectées RF0 ; RF1 est en deçà de la plage temporelle prescrite BIP ; BSP ou si le temps de présence mesuré d'oscillations détectées RF1 est inférieur au temps minimum d'oscillations TMRH.
- on remplace le temps mesuré RF0 d'absence d'oscillations détectées par la somme des temps mesurés d'absence et de présence d'oscillations détectées RF0 ; RF1, puis
- on remet à 0 le temps mesuré de présence d'oscillations détectées RF1.

Si la somme des temps mesurés d'absence et de présence d'oscillations détectées RF0 ; RF1 est au-delà de la plage temporelle prédéterminée BIP ; BSP ou d'un temps maximal prédéterminé TCMax, on remet à 0 chacun des temps mesurés d'absence et de présence d'oscillations détectées RF0 ; RF1.

Si les deux conditions mentionnées ci-dessus sur la somme des temps de présence et d'absence RF1, RF0 et sur le temps de présence RF1 ne sont pas réalisées, on remet à 0 chacun des temps mesurés d'absence et de présence d'oscillations détectées RF0 ; RF1.

Dans une réalisation, le temps maximal prédéterminé TCMax est sensiblement égal à deux fois le temps TCM de cycle respiratoire moyen sur les trois derniers cycles mesurés.

La plage temporelle prescrite BIP ; BSP est sensiblement comprise entre 10 % et 120 % du temps de cycle moyen calculé TCM.

Le temps minimum d'oscillation TMRH est sensiblement égal à 7 % du temps de cycle moyen calculé TCM.

Le temps d'attente prescrit TAR est compris entre 1 et 30 minutes et est par exemple sensiblement égal à 1 minute.

La huitième commande C8 d'augmentation de pression est comprise entre 0,1 mbar et 10 mbar et est par exemple sensiblement égale à 1 mbar.

La plage P1 de fréquence de détection d'oscillations est comprise entre sensiblement 30 et 300 Hz.

On mémorise la chronologie des événements détectés et on relève, par exemple après une nuit, la chronologie mémorisée.

A cet effet, l'unité centrale U de l'appareil comporte une mémoire non représentée pouvant être écrite et lue avec la chronologie des événements détectés.

Cette chronologie peut être visualisée par exemple sur un moniteur en lisant le contenu de la mémoire, par l'intermédiaire d'un ordinateur non représenté.

## Revendications

1. Appareil de fourniture de pression d'air à un patient souffrant de troubles du sommeil tels qu'apnée comportant une unité (U) centrale de traitement et de commande de pression, un module (MPD) commandé de fourniture de pression, un masque (MVA) pour voies aériennes supérieures du patient, un conduit (CF) de fourniture de pression d'air du module (MPD) au masque (MVA), un capteur (CDAF) de débit d'air fourni, relié à l'unité centrale (U) et un capteur (CPM) de pression dans le masque (MVA), relié à l'unité centrale (U), un logiciel étant intégré à l'unité centrale (U), le logiciel étant agencé pour
- mesurer la pression d'air dans le masque et le débit d'air fourni au masque
- déterminer à partir des variables mesurées si des événements représentatifs de troubles du sommeil apparaissent ou non,
**caractérisé en ce que** le logiciel est agencé pour :
- pendant chacun de plusieurs (NINT) intervalles de temps (TAC(j)) consécutifs prédéterminés d'une période prédéterminée (PDAC) de détection d'apnée,
• détecter les oscillations de la courbe de débit mesurée, qui sont de fréquences comprises dans une plage (P2) de fréquence, et détecter si l'amplitude des oscillations détectées de la courbe de débit mesurée passe successivement au-dessus puis au-dessous d'un premier seuil (SAC) prédéterminé d'apnée centrale ou si cette amplitude reste inférieure au premier seuil (SAC) d'apnée centrale,
• en présence d'au moins une détection d'un passage au-dessus puis au-dessous du premier seuil (SAC), on compte une détection (CAC(D)) d'apnée centrale ;
- à chaque période (PDAC) de détection d'apnée,
• faire la somme (SIG) des nombres (CAC(i)) de détections d'apnée centrale comptées, successivement sur les (D+1) dernières périodes de détection d'apnée,
• commander (C2) une augmentation prédéterminée de pression d'air délivré si la somme (SIG) des nombres (CAC(i)) de détections comptées est inférieure ou égale à un deuxième seuil (SQAC) prédéterminé de qualification d'apnée centrale ;
• commander un maintien de pression d'air délivré, si la somme (SIG) des nombres (CAC(i)) de détections comptées est supérieure au deuxième seuil (SQAC).

2. Appareil selon la revendication 1, **caractérisé en ce que** le deuxième seuil (SQAC) de qualification d'apnée centrale est compris entré 0 et 50, et est par exemple sensiblement égal à 10, les intervalles de temps (TAC(j)) consécutifs prédéterminés correspondent à dix (NINT) intervalles de temps consécutifs de chacun sensiblement 100 ms, la période (PDAC) de détection d'apnée correspondant sensiblement à 1 s,
la deuxième commande (C2) d'augmentation de pression est comprise entre 1 et 10 mbar et est par exemple sensiblement égale à + 1 mbar,
le nombre (D+1) de périodes (PDAC) de détection d'apnée, sur lesquelles le logiciel fait la somme des nombres (CAC(i)) comptés de détection d'apnée centrale est sensiblement égal à 5,
la deuxième plage (P2) de fréquence d'oscillations est comprise entre sensiblement 2.5 et 47 Hz.

3. Appareil selon l'une quelconque des revendications 1 et 2, **caractérisé en ce que** les nombres (CAC(i)) comptés de détections d'apnée centrale sont remis à 0 lors de la mise en marche de l'appareil.

4. Appareil l'une quelconque des revendications 1 à 3, **caractérisé en ce que** le logiciel est agencé pour :
- calculer à chaque fin de cycle respiratoire mesuré, l'amplitude moyenne (AM) sur un quatrième nombre prédéterminé (Y4) de cycles respiratoires précédents,
- si l'amplitude mesurée du dernier cycle respiratoire est inférieure à l'amplitude moyenne (AM) calculée multipliée par un premier facteur d'hypopnée prédéterminé (FHO), alors ajouter à un compteur (CTHO) de temps en hypopnée la durée (TC) du dernier cycle respiratoire mesuré,
• si la valeur actuelle du compteur de temps en hypopnée (CTHO) est supérieure ou égale à un temps minimum d'hypopnée (TMHO), commander (C5) une cinquième augmentation prédéterminée de pression,
• après la fin d'un cinquième nombre prédéterminé (Y5) de cycles respiratoires suivant la cinquième commande (C5) d'augmentation de pression, commander (C6) une sixième augmentation prédéterminée de pression
• après la fin d'un sixième nombre prédéterminé (Y6) de cycles respiratoires, supérieur au cinquième nombre (Y5), suivant la cinquième commande (C5) d'augmentation de pression, commander (C7) une septième augmentation de pression,
- le compteur de temps en hypopnée (CTHO) étant initialisé à 0 lors de la mise en

5. Appareil selon la revendication 4, **caractérisé en ce que** :
- le quatrième nombre déterminé (Y4) de cycles respiratoires de calcul d'amplitude moyenne est sensiblement égal à 8,
le premier facteur prédéterminé (FHO) d'hypopnée est compris entre 1 et 100 % et est par exemple sensiblement égal à 40 %,
le temps minimum d'hypopnée (TMHO) est compris entre 1 s et 25 s et est par exemple sensiblement égal à 10 s.
les cinquième et sixième nombres prédéterminés (Y5 ; Y6) de cycles respiratoires sont sensiblement égaux à respectivement 2 et 4,
la cinquième augmentation (C5) prédéterminée de pression est comprise entre 0,1 mbar et 10 mbar et est par exemple sensiblement égale à + 1 mbar,
les sixième et septième augmentations (C6 ; C7) prédéterminées de pression étant chacune inférieures à la cinquième commande (C5) et étant par exemple chacune sensiblement égales à la moitié de la cinquième augmentation (C5) de pression.

6. Appareil selon la revendication 5, **caractérisé en ce que** le logiciel est agencé pour :
- si l'amplitude mesurée du dernier cycle respiratoire est supérieure ou égale à l'amplitude moyenne (AM) calculée multipliée par la premier facteur d'hypopnée (FHO), alors calculer le temps (TCM) de cycles respiratoires moyen sur un septième nombre prédéterminé (Y7) de cycles précédents ;
• si la durée mesurée (TC) du dernier cycle est supérieure à un huitième nombre prédéterminé (Y8) multiplié par le temps de cycle respiratoire moyen calculé (TCM), ajouter au compteur de temps en hypopnée (CTHO) la durée (TC) mesurée du dernier cycle, multipliée par un deuxième facteur (F2) d'hypopnée,
• si l'amplitude mesurée du dernier cycle respiratoire mesuré est supérieure à un troisième facteur (F3) d'hyperventilation, supérieur au premier facteur (FHO) d'hypopnée, multiplié par l'amplitude moyenne calculée (AM), qualifier le dernier cycle d'hyperventilé, on incrémente d'une unité un compteur de cycles hyperventilés (CCH), remettre à 0 un compteur (CCN) de cycles à ventilation normale et
• si la valeur du compteur (CCH) de cycles hyperventilés est supérieure ou égale à un neuvième nombre prédéterminé (Y9),
• si la durée du dernier cycle (TC) est supérieure ou égale au huitième nombre (Y8) multiplié par le temps de cycle moyen calculé (TCM), ajouter au compteur (CTHO) de temps en hypopnée le deuxième facteur (F2) multiplié par la durée du dernier cycle respiratoire (TC),
• et sinon, remettre à 0 le compteur (CTHO) de temps en hypopnée ;
puis remettre à 0 un compteur (CCHO) de cycles hypoventilés et on calcule l'amplitude moyenne (AM) de cycle respiratoire sur le nombre prédéterminé (Y4) de cycles respiratoires précédents,
• si l'amplitude mesurée du dernier cycle respiratoire mesuré est inférieure ou égale au troisième facteur (F3) multiplié par l'amplitude moyenne calculée (AM), qualifier le dernier cycle de cycle à ventilation normale, remettre à 0 le compteur (CCH) de cycles hyperventilés et incrémenter d'une unité le compteur (CCN) de cycles à ventilation normale, et
■ si la valeur du compteur (CCN) de cycles à ventilation normale est supérieure ou égale à un dixième nombre (Y10) prédéterminé,
■ si la durée du dernier cycle (TC) est supérieure ou égale au huitième nombre (Y8) multiplié par le temps de cycle moyen calculé (TCM), affecter au compteur (CTHO) de temps en hypopnée le deuxième facteur (F2) multiplié par la durée du dernier cycle (TC) et remettre à 0 le compteur (CCN) de cycle à ventilation normale,
■ et sinon, remettre à 0 le compteur (CTHO) de temps en hypopnée ;
puis remettre à 0 le compteur (CCHO) de cycles hypoventilés et calculer l'amplitude moyenne du cycle respiratoire sur le nombre prédéterminé (Y4) de cycles respiratoires.

7. Appareil selon la revendication 6, **caractérisé en ce que**
le deuxième facteur (F2) est sensiblement égal à 5/8,
le troisième facteur d'hyperventilation (F3) est compris entre 100 % et 200 % et est par exemple sensiblement égal à 140 %,
les septième, huitième, neuvième et dixième nombres prédéterminés (Y7 ; Y8 ; Y9 ; Y10) sont respectivement sensiblement égaux à 3 ; 2 ; 2 ; et 2.

8. Appareil selon l'une quelconque des revendications 1 à 7, **caractérisé en ce que** :
- si la pression mesurée (P) est inférieure à une valeur de pression haute (PH) prédéterminée, remettre à 0 un compteur de temps en pression haute (TPH) ;
- si la valeur du compteur de temps en pression haute (TPH) est supérieure à un temps maximum de pression haute (TMPH) et
• si la valeur maximale de pression réglée (Pmaxi) est inférieure à une valeur de • pression de sécurité (PSEC) prédéterminée, commander la pression (P) à cette valeur maximale de pression réglée (Pmaxi);
• si la valeur minimale de pression réglée (Pmini) est supérieure à une valeur de pression de sécurité (PSEC) prédéterminée, commander la pression (P) à cette valeur minimale de pression réglée (Pmini);
• si les deux précédentes conditions ne sont pas réalisées, commander la pression (P) à la valeur de pression de sécurité (PSEC) ;
puis
• remettre à 0 le compteur de temps en pression haute (TPH).

9. Appareil selon la revendication 8, **caractérisé en ce que** la valeur de pression haute (PH) est comprise entre 10 mbar et 25 mbar et est par exemple sensiblement égale à 17 mbar,
le temps maximum de pression haute (TMPH) est compris entre 1 et 100 minutes et est par exemple sensiblement égal à 10 minutes ou 30 minutes,
la valeur de pression de sécurité (PSEC) est sensiblement égale à 8 mbar.

10. Appareil selon l'une quelconque des revendications 1 à 9, **caractérisé en ce que** le logiciel est agencé pour : mesurer une fuite d'air, sensiblement-égale au débit moyen pendant la respiration du patient,
- si la fuite mesurée d'air est supérieure à un niveau prédéterminé de fuite (NFM), invalider les commandes d'augmentation de pression.

11. Appareil selon la revendication 10, **caractérisé en ce que** le niveau prédéterminé de fuite (NFM) est sensiblement égal à un coefficient (A) de fuite multiplié par une pression d'air filtrée dans le masque, ajouté à un coefficient (B) additif de fuite, le coefficient (A) de fuite étant compris entre 0 et 10 l/minute.mbar et étant par exemple sensiblement égal à 2,5 l/minute.mbar, et
le coefficient (B) additif de fuite est compris entre 0 et 100 I/mn et est par exemple sensiblement égal à 50 l/mn.

12. Appareil selon l'une quelconque des revendications 1 à 11, **caractérisé en ce que** le logiciel est agencé pour :
- détecter la courbe de pression mesurée présente des oscillations, telles que de vibrations acoustiques, comprises dans une plage (P1) de fréquence,
- mesurer le temps (RF1) de présence d'oscillations détectées entre deux absences successives d'oscillations détectées et le temps (RF0) d'absence d'oscillations détectées entre deux présences successives d'oscillations détectées ;
- si la somme des temps mesurés d'absence et de présence d'oscillations détectées (RF0 ; RF1) est comprise dans une plage temporelle prescrite (BIP ; BSP),
- si le temps de présence d'oscillations (RF1) mesuré est supérieur ou égal à un temps minimum d'oscillations (TMRH) et
- si la valeur d'un compteur (CTAR) de temps écoulé depuis l'avant-demière fois que les conditions temporelles précédentes ont été réalisées, est supérieure à un temps d'attente prescrit (TAR), commander (C8) une huitième augmentation prédéterminée de pression et remettre le compteur de temps écoulé (CTAR) à 0.

13. Appareil suivant la revendication 12, **caractérisé en ce que** le logiciel est agencé pour :
- si la somme des temps mesurés de présence et d'absence d'oscillations détectées (RF0 ; RF1) est en deçà de la plage temporelle prescrite (BIP ; BSP) ou si le temps de présence mesuré d'oscillations détectées (RF1) est inférieur au temps minimum d'oscillations (TMRH).
• remplacer le temps mesuré (RF0) d'absence d'oscillations détectées par la somme des temps mesurés d'absence et de présence d'oscillations détectées (RF0 ; RF1), puis
• remettre à 0 le temps mesuré de présence d'oscillations détectées (RF1), et
- si la somme des temps mesurés d'absence et de présence d'oscillations détectées (RF0 ; RF1) est au-delà de la plage temporelle prédéterminée (BIP ; BSP) ou d'un temps maximal prédéterminé (TCMax), remettre à 0 chacun des temps mesurés d'absence et de présence d'oscillations détectées (RF0 ; RF1) ; et sinon
- remettre à 0 chacun des temps mesurés d'absence et de présence d'oscillations détectées (RF0 ; RF1).

14. Appareil selon l'une quelconque des revendications 12 et 13, **caractérisé en ce que** :
le temps maximal prédéterminé est sensiblement égal à deux fois le temps (TCM) de cycle respiratoire moyen sur les trois derniers cycles mesurés ;
- la plage temporelle prescrite (BIP ; BSP) est sensiblement comprise entre 10 % et 120 % du temps de cycle moyen calculé (TCM) ;
- le temps minimum d'oscillation (TMRH) est sensiblement égal à 7 % du temps de cycle moyen calculé (TCM);
- le temps d'attente prescrit (TAR) est compris entre 1 et 30 minutes et est par exemple sensiblement égal à 1 minute ;
- la huitième commande (C8) d'augmentation de pression est comprise entre 0,1 mbar et 10 mbar et est par exemple sensiblement égale à 1 mbar ;
- la plage (P1) de fréquence de détection d'oscillations est comprise entre sensiblement 30 et 300 Hz.

15. Appareil suivant l'une quelconque des revendications 1 à 14, **caractérisé en ce que** le logiciel est agencé pour : mémoriser la chronologie des événements détectés et relever, par exemple après une nuit, la chronologie mémorisée.

16. Appareil selon l'une quelconque des revendications 1 à 15 en ce qu'il comporte une mémoire de la chronologie des événements détectés, dont le contenu est apte à être relevé, par exempte après une nuit.

## Claims

1. Device for supplying air pressure to a patient suffering from sleep disturbances such as apnoea comprising a central unit (U) for processing and pressure control, a controlled module (MPD) for supplying pressure, a mask (MVA) for the patient's upper airways, a pipe (CF) for supplying the air pressure from the module (MPD) to the mask (MVA), a sensor (CDAF) of the flow of air supplied, connected to the central unit (U), and a sensor (CPM) of the pressure in the mask (MVA), connected to the central unit (U), the central unit (U) being equipped with a software program, the software being designed such as to:
- measure the air pressure in the mask and the flow of air supplied to the mask;
- determine, based on measured variables, whether or not any events that are representative of sleep disturbances are found,
**characterised in that** the software is designed such as:
- during each one of several (NINT) predetermined consecutive time intervals (TAC(j)) of a predetermined apnoea detection period (PDAC),
- to detect oscillations in the measured flow curve, which are frequencies comprised within a range (P2) of frequencies, and to detect whether the amplitude of the detected oscillations in the measured flow curve successively passes above and under a first predetermined threshold (SAC) for central apnoea or if this amplitude remains below the first threshold (SAC) for central apnoea,
- when it detects the passage above and then under the first threshold (SAC), to count a detection (CAC(D)) of central apnoea;
- during each apnoea detection period (PDAC),
- to add up (SIG) the number (CAC(i)) of central apnoea detections counted, consecutively in the (D + 1) last apnoea detection periods,
- to command (C2) a predetermined increase in the air pressure delivered if the sum (SIG) of the numbers (CAC(i)) of detections counted is less than or equal to a second predetermined threshold (SQAC) for qualifying as central apnoea;
- to command keeping up the delivered air pressure, if the sum (SIG) of the numbers (CAC(i)) of detections counted is higher than the second threshold (SQAC).

2. Device according to claim 1, **characterised in that** the second threshold (SQAC) for qualifying as central apnoea is comprised between 0 and 50 and is, for example, approximately equal to 10,
the predetermined consecutive time intervals (TAC(j)) correspond to ten (NINT) consecutive time intervals of approximately 100 ms each, the period (PDAC) for detection of apnoea corresponding to approximately 1 s,
the second command (C2) to increase the pressure is comprised between 1 and 10 mbar and is, for example, approximately equal to + 1 mbar,
the number (D + 1) of apnoea detection periods (PDAC) in which the software calculates the sum of the counted numbers (CAC(i)) for detection of central apnoea is approximately equal to 5,
the second range (P2) of oscillation frequencies is comprised approximately between 2.5 and 47 Hz.

3. Device according to either one of the claims 1 and 2, **characterised in that** the counted numbers (CAC(i)) of central apnoea detections are reset to 0 when the device is switched on.

4. Device according to any one of the claims from 1 to 3, **characterised in that** the software is designed such as:
- to calculate, at the end of each breathing cycle measured, the average amplitude (AM) over a fourth predetermined number (Y4) of preceding breathing cycles,
- if the measured amplitude of the last breathing cycle is less than the average amplitude (AM) calculated multiplied by a first predetermined oligopnea factor (FHO), then to add the duration (TC) of the last breathing cycle measured to an oligopnea duration counter (CTHO),
- if the current value of the oligopnea duration counter (CTHO) is higher than or equal to a minimum oligopnea duration (TMHO), to command (C5) a fifth predetermined pressure increase,
- after the end of a fifth predetermined number (Y5) of breathing cycles following the fifth command (C5) to increase the pressure, to command (C6) a sixth predetermined pressure increase;
- after the end of a sixth predetermined number (Y6) of breathing cycles, which is higher than the fifth number (Y5), following the fifth command (C5) to increase the pressure, to command (C7) a seventh pressure increase,
- the oligopnea duration counter (CTHO) being reset to zero when the device is switched on.

5. Device according to claim 4, **characterised in that**:
- the fourth predetermined number (Y4) of breathing cycles for calculating the average amplitude is approximately equal to 8,
the first predetermined oligopnea factor (FHO) is comprised between 1 and 100% and is, for example, approximately equal to 40%,
the minimum oligopnea duration (TMHO) is comprised between 1 s and 25 s and is, for example, approximately equal to 10 s.
the fifth and sixth predetermined numbers (Y5; Y6) of breathing cycles are approximately equal to 2 and 4 respectively,
the fifth predetermined pressure increase (C5) is comprised between 0.1 mbar and 10 mbar and is, for example, approximately equal to + 1 mbar,
the sixth and seventh predetermined pressure increases (C6, C7) each being lower than the fifth command (C5) and each being, for example, approximately equal to half the fifth pressure increase (C5).

6. Device according to claim 5, **characterised in that** the software is designed such as:
- if the measured amplitude of the last breathing cycle is higher than or equal to the calculated average amplitude (AM) multiplied by the first oligopnea factor (FHO), then to calculate the average breathing cycle duration (TCM) over a seventh predetermined number (Y7) of preceding cycles;
- if the measured duration (TC) of the last cycle is higher than an eighth predetermined number (Y8) multiplied by the calculated average breathing cycle duration (TCM), to add the measured duration (TC) of the last cycle, multiplied by a second oligopnea factor (F2) to the oligopnea duration counter (CTHO),
- if the measured amplitude of the last breathing cycle measured is higher than a third hyperventilation factor (F3), which is higher than the first oligopnea factor (FHO), multiplied by the calculated average amplitude (AM), to qualify the last cycle as hyperventilated, to increase a counter of hyperventilation cycles (CCH) by one unit and to reset a normal ventilation cycle counter (CCN) to 0 and
- if the value of the hyperventilated cycle counter (CCH) is higher than or equal to a ninth predetermined number (Y9),
- if the duration of the last cycle (TC) is higher than or equal to the eighth number (Y8) multiplied by the calculated average cycle duration (TCM), to add the second factor (F2) multiplied by the duration of the last breathing cycle (TC) to the oligopnea duration counter (CTHO),
- and if not, to reset the oligopnea duration counter (CTHO) to 0;
then to reset a hyperventilated cycle counter (CCHO) to 0 and to calculate the average amplitude (AM) of breathing cycles over the predetermined number (Y4) of preceding breathing cycles,
- if the measured amplitude of the last breathing cycle measured is less than or equal to the third factor (F3) multiplied by the calculated average amplitude (AM), to qualify the last cycle as a normal ventilation cycle, to reset the hyperventilated cycle counter (CCH) to 0 and to increase the normal ventilation cycle counter (CCN) by one unit, and
- if the value of the normal ventilation cycle counter (CCN) is higher than or equal to a tenth predetermined number (Y10),
- if the duration of the last cycle (TC) is higher than or equal to the eighth number (Y8) multiplied by the calculated average cycle duration (TCM), to allocate the second factor (F2) multiplied by the duration of the last cycle (TC) to the oligopnea duration counter (CTHO) and to reset the normal ventilation cycle counter (CCN) to 0,
- and if not, to reset the oligopnea duration counter (CTHO) to 0;
then, to reset the hyperventilated cycle counter (CCHO) to 0 and to calculate the average amplitude of the breathing cycle over the predetermined number (Y4) of breathing cycles.

7. Device according to claim 6, **characterised in that**
the second factor (F2) is approximately equal to 5/8,
the third hyperventilation factor (F3) is comprised between 100% and 200% and is, for example, approximately equal to 140%,
the seventh, eighth, ninth and tenth predetermined numbers (Y7; Y8; Y9; Y10) are approximately equal to 3; 2; 2 and 2 respectively.

8. Device according to any one of the claims from 1 to 7, **characterised in that** the software is designed such as:
- if the pressure measured (P) is lower than a predetermined high-pressure value (PH), to reset the high-pressure duration counter (TPH) to 0;
- if the value of the high-pressure duration counter (TPH) is higher than maximum high-pressure duration (TMPH) and
- if the maximum adjusted pressure value (Pmaxi) is lower than a predetermined safe pressure value (PSEC), to adjust the pressure (P) to this maximum adjusted pressure value (Pmaxi) ;
- if the minimum adjusted pressure value (Pmini) is higher than a predetermined safe pressure value (PSEC), to adjust the pressure (P) to this minimum adjusted pressure value (Pmini) ;
- if the two preceding conditions are not fulfilled, to adjust the pressure (P) to the safe pressure value (PSEC);
and then
- to reset the value of the high-pressure duration counter to 0 (TPH).

9. Device according to claim 8, **characterised in that** the high-pressure value (PH) is comprised between 10 mbar and 25 mbar and is, for example, approximately equal to 17 mbar,
the maximum high-pressure duration (TMPH) is comprised between 1 and 100 minutes and is, for example, approximately equal to 10 minutes or 30 minutes,
the safe pressure value (PSEC) is approximately equal to 8 mbar.

10. Device according to any one of the claims from 1 to 9, **characterised in that** the software is designed such as to measure an air leakage, approximately equal to the average flow during the patient's breathing,
- if the measured air leakage is higher than a predetermined leakage level (NFM), to invalidate the pressure increase commands.

11. Device according to claim 10, **characterised in that** the predetermined leakage level (NFM) is approximately equal to a leakage coefficient (A) multiplied by an air pressure filtered in the mask, added to a leakage addition coefficient (B), the leakage coefficient (A) being comprised between 0 and 10 l/minute-mbar and being, for example, approximately equal to 2.5 l/minute-mbar, and
the leakage addition coefficient (B) is comprised between 0 and 100 l/min and is, for example, approximately equal to 50 l/min.

12. Device according to any one of the claims 1 to 11, **characterised in that** the software is designed such as:
- to detect whether the measured pressure curve has oscillations such as acoustic vibrations, comprised within a range (P1) of frequencies,
- to measure the time (RF1) during which oscillations are detected between two successive absences of oscillations detected and the time (RF0) during which no oscillations are detected between two successive oscillations detected;
- if the sum of the measured times of absence and presence of oscillations detected (RF0; RF1) is comprised within a stipulated range of times (BIP; BSP),
- if the time of presence of oscillations (RF1) measured is higher than or equal to a minimum oscillation duration (TMRH) and
- if the value of a counter (CTAR) of time elapsed since the last but one time the preceding time conditions were fulfilled is higher than a stipulated waiting time (TAR), to command an eighth predetermined pressure increase (C8) and to reset the elapsed time counter (CTAR) to 0.

13. Device according to claim 12, **characterised in that** the software is designed such as:
- if the sum of the measured times of absence and presence of oscillations detected (RF1; RF0) is less than the stipulated range of times (BIP; BSP) or if the measured time of presence of oscillations detected (RF1) is less than the minimum oscillation duration (TMRH),
- to replace the measured time (RF0) of absence of oscillations detected with the sum of the measured times of absence and presence of oscillations detected (RF0; RF1), then
- to reset the measured time of the presence of oscillations detected (RF1) to 0, and
- if the sum of the measured times of absence and presence of oscillations detected (RF0; RF1) is more than the stipulated range of times (BIP; BSP) or a predetermined maximum time (TCMax), to reset each of the measured times of absence and presence of oscillations detected (RF0; RF1) to 0; and if not
- to reset each of the measured times of absence and presence of oscillations detected (RF0; RF1) to 0.

14. Device according to any one of the claims 12 and 13, **characterised in that**:
the predetermined maximum time is approximately equal to twice the time (TCM) of the average breathing cycle over the last three measured cycles;
- the stipulated time range (BIP; BSP) is comprised approximately between 10% and 120% of the calculated average cycle duration (TCM);
- the minimum oscillation duration (TMRH) is approximately equal to 7% of the calculated average cycle duration (TCM);
- the stipulated waiting time (TAR) is comprised between 1 and 30 minutes and is, for example, approximately equal to 1 minute;
- the eighth command (C8) to increase the pressure is comprised between 0.1 mbar and 10 mbar and is, for example, approximately equal to 1 mbar;
- the range (P1) of frequencies for detecting oscillations is comprised approximately between 30 and 300 Hz.

15. Device according to any one of the claims 1 to 14, **characterised in that** the software is designed such as to memorise the chronology of the events detected and to download, after one night for example, the memorised chronology.

16. Device according to any one of the claims from 1 to 15, **characterised in that** it comprises a memory of the chronology of the events detected, the contents of which are capable of being downloaded, for example after one night.

## Patentansprüche

1. Gerät zur Bereitstellung von Pressluft für einen an Schlafstörungen, wie z. B. Apnoe, leidenden Patienten, mit einer Zentraleinheit (U) und einer Drucksteuerung, einem gesteuerten Modul für die Bereitstellung von Druck (MPD), einer Maske (MVA) für die oberen Luftwege des Patienten, einer Leitung zur Bereitstellung von Luftdruck des Moduls (MPD) an die Maske (MVA), einen mit der Zentraleinheit (U) verbundenen Drucksensor (CDAF) der bereitgestellten Luft, und einem mit der Zentraleinheit (U) verbundenen Drucksensor (CPM) in der Maske (MVA), einer in die Zentraleinheit (U) integrierten Software, wobei die Software angeordnet ist, um
- den Luftdruck in der Maske und den der Maske bereitgestellten Luftdurchsatz zu messen,
- ausgehend von den gemessenen Variablen zu bestimmen, ob die die Schlafstörungen darstellenden Ereignisse auftreten oder nicht,
**dadurch gekennzeichnet, dass** die Software angeordnet ist, um:
- während jedes von mehreren (NINT) aufeinander folgenden, vorbestimmten Zeitintervallen (TAC(j)) einer vorbestimmten Periode (PDAC) zur Feststellung der Apnoe
- die Schwankungen der gemessenen Durchsatzkurve festzustellen, die in einem Frequenzbereich (P2) inbegriffen Frequenzen sind und festzustellen, ob die Amplitude der festgestellten Schwankungen der gemessenen Durchsatzkurve nacheinander einen vorbestimmten ersten Schwellenwert (SAC) einer zentralen Apnoe über- oder unterschreitet oder ob diese Amplitude unterhalb des ersten Schwellenwertes (SAC) einer zentralen Apnoe bleibt,
* bei Vorhandensein wenigstens einer Feststellung eines Überschreitens und dann eines Unterschreitens des ersten Schwellenwertes (SAC), eine Feststellung (CAC(D)) einer zentralen Apnoe zu zählen;
- bei jeder Periode (PDAC) einer festgestellten Apnoe,
die Summe (SIG) der Anzahlen (CAC(j)) von gezählten Feststellungen einer zentralen Apnoe zu bilden, und zwar aufeinander folgend für die letzten Feststellungsperioden einer Apnoe (D+1),
* eine vorbestimmte Erhöhung des bereitgestellten Luftdrucks zu steuern (C2), wenn die Summe (SIG) der Anzahlen (CAC (i)) von gezählten Feststellungen unterhalb von oder gleich einem zweiten vorbestimmten Qualifizierungsschwellenwert (SQAC) einer zentralen Apnoe liegt,
* eine Aufrechterhaltung des bereitgestellten Luftdrucks zu steuern, wenn die Stumme (SIG) der Anzahlen (CAC(i)) von gezählten Feststellungen höher als der zweite Schwellenwert (SQAC) ist.

2. Gerät gemäß Anspruch 1, **dadurch gekennzeichnet, dass** der zweite Qualifizierungsschwellenwert (SQAC) einer zentralen Apnoe zwischen 0 und 50 inbegriffen ist und z. B. deutlich gleich 10 ist,
die vorbestimmten, aufeinander folgenden Zeitintervalle (TAC(j)) zehn aufeinander folgenden Zeitintervallen (NINT) von jeweils deutlich 100 msek entsprechen, wobei die Feststellungsperiode (PDAC) der Apnoe deutlich 1 Sek. entspricht,
die zweite Steuerung (C2) der Druckerhöhung zwischen 1 und 10 mbar inbegriffen ist und z. B. deutlich gleich + 1 mbar ist,
die Anzahl (D+1) von Feststellungsperioden (PDAC) einer Apnoe, aus denen die Software die Summe der gezählten Anzahlen (CAC(j)) zur Feststellung einer zentralen Apnoe bildet, deutlich gleich 5 ist,
der zweite Schwankungs-Frequenzbereich (P2) zwischen deutlich 2,5 und 47 Hz inbegriffen ist.

3. Gerät gemäß Anspruch 1 und 2, **dadurch gekennzeichnet, dass** die gezählten Anzahlen (CAC(i)) einer zentralen Apnoe beim Einschalten des Geräts wieder auf 0 gesetzt werden.

4. Gerät gemäß Anspruch 1 bis 3, **dadurch gekennzeichnet, dass** die Software angeordnet ist, um:
- bei jedem gemessenen Ende eines Atmungszyklus die durchschnittliche Amplitude (AM) auf einer vorbestimmten vierten Anzahl (Y4) von vorbestimmten Atmungszyklen zu berechnen,
- wenn die gemessene Amplitude des letzten Atmungszyklus unterhalb der mit einem ersten vorbestimmten Hypopnoe-Faktor (FHO) multiplizierten, berechneten durchschnittlichen Amplitude liegt, die Dauer (TC) des letzten gemessenen Atmungszyklus dann einem Zeitzähler (CTHO) der Hypopnoe hinzuzufügen,
* wenn der aktuelle Wert des Zeitzählers der Hypopnoe (CTHO) höher als oder gleich einer Mindest-Hypopnoe-Zeit (TMHO) ist, eine fünfte, vorbestimmte Druckerhöhung zu steuern (C5),
* nach dem Ende einer fünften Anzahl (Y5) von Atmungszyklen im Anschluss an die fünfte Steuerung (C5) der Druckerhöhung, eine sechste vorbestimmte Druckerhöhung (C6) zu steuern;
* Nach dem Ende eines sechsten vorbestimmten Atmungszyklus (Y6) über der fünften Anzahl (Y5) im Anschluss an die fünfte Steuerung (C5) einer Druckerhöhung, eine siebte Druckerhöhung (C7) zu steuern,
- wobei der Zeitzähler in Hypopnoe (CTHO) beim Einschalten des Geräts bei 0 initialisiert wird.

5. Gerät gemäß Anspruch 4, **dadurch gekennzeichnet, dass**:
- die vierte bestimmte Anzahl (Y4) von Atmungszyklen der Berechnung der durchschnittlichen Amplitude deutlich gleich 8 ist,
der erste vorbestimmte Hypopnoe-Faktor (FHO) zwischen 1 und 100 % inbegriffen ist und z. B. deutlich gleich 40 % ist,
die Mindest-Hypopnoe-Zeit (TMHO) zwischen 1 und 25 Sek. inbegriffen ist und z. B. deutlich gleich 10 Sek. ist,
die fünften und sechsten vorbestimmten Anzahlen (Y5; Y6) von Atmungszyklen jeweils deutlich gleich 2 und 4 sind,
die fünfte vorbestimmte Druckerhöhung (C5) zwischen 0,1 mbar und 10 mbar inbegriffen ist und z. B. deutlich gleich + 1 mbar ist,
wobei die sechsten und siebten vorbestimmten Druckerhöhungen (C6; C7) jeweils unterhalb der fünften Steuerung (C5) liegen und zum Beispiel jeweils deutlich gleich der Hälfte der fünften Druckerhöhung (C5) sind.

6. Gerät gemäß Anspruch 5, **dadurch gekennzeichnet, dass** die Software angeordnet ist, um:
- wenn die gemessene Amplitude des letzten Atmungszyklus größer als oder gleich der durchschnittlichen mit ersten Hypopnoe-Faktor multiplizierten (FHO) gemessenen Amplitude (AM) ist, dann die durchschnittliche Zeit der Atmungszyklen auf einer siebten vorbestimmten Anzahl (Y7) von vorherigen Zyklen zu berechnen;
* wenn die gemessene Dauer (TC) des letzten Zyklus größer ist als ein Achtel einer vorbestimmten Anzahl (Y8), multipliziert mit der durchschnittlichen Zeit der Atmungszyklen (TCM), die gemessene Dauer (TC) des letzten Zyklus, multipliziert mit einem zweiten Hypopnoe-Faktor (F2) dem Zeitzähler der Hypopnoe (CTHO) hinzuzufügen,
* wenn die gemessene Amplitude des letzten gemessenen Atmungszyklus größer ist als ein dritter Hyperventilationsfaktor (F3), der größer ist als ein erster Hypopnoefaktor (FHO), multipliziert mit der durchschnittlichen berechneten Amplitude (AM), den letzten hyperventilierten Zyklus zu qualifizieren, wird ein Zähler hyperventilierter Zyklen (CCH) um eine Einheit inkrementiert und ein Zähler (CCN) für Zyklen mit normaler Ventilation wird wieder auf 0 gesetzt und
* wenn der Wert des Zählers (CCH) der hyperventilierten Zyklen größer als oder gleich einem Neuntel der vorbestimmten Anzahl (Y9) ist
* wenn die Dauer des letzten Zyklus (TC) größer als oder gleich dem Achtel der Anzahl (Y8) multipliziert mit der durchschnittlichen Zykluszeit (TCM) ist, dem Zeitzähler (CTHO) der Hypopnoe den zweiten Faktor (F2) multipliziert mit der Dauer des letzten Atmungszyklus (TC) hinzufügen,
* und andernfalls den Zeitzähler (CTHO) in Hypopnoe wieder auf 0 zu setzen;
anschließend einen Zähler (CCHO) der hyperventilierten Zyklen wieder auf 0 zu setzen und die durchschnittliche Amplitude (AM) des Atmungszyklus auf der vorbestimmten Anzahl (Y4) von vorherigen Atmungszyklen zu berechnen,
* wenn die gemessene Amplitude des gemessenen Atmungszyklus kleiner als oder gleich dem dritten Faktor (F3) multipliziert mit der durchschnittlichen berechneten Amplitude (AM) ist, den letzten Zyklus mit normaler Ventilation zu berechnen, den Zähler (CCH) der hyperventilierten Zyklen wieder auf 0 setzen und den Zähler (CCN) der Zyklen mit normaler Ventilation (CCN) um eine Einheit zu inkrementierten und
* wenn der Wert des Zähler (CCN) der Zyklen mit normaler Ventilation größer als oder gleich einem Zehntel der vorbestimmten Anzahl (Y10) ist,
* wenn die Dauer des letzten Zyklus (TC) größer als oder gleich dem Achtel der Anzahl (Y8) ist, multipliziert mit der Zeit des durchschnittlichen berechneten Zyklus (TCM) dem Zeitzähler (CTHO) in Hypopnoe den zweiten Faktor (F2) multipliziert mit der Dauer des letzten Zyklus (TC) hinzufügen und den Zähler (CCN) der Zyklen mit normaler Ventilation wieder auf 0 setzen;
* und andernfalls den Zeitzähler (CTHO) in Hypopnoe wieder auf 0 setzen;
dann den Zähler (CCHO) der hyperventilierten Zyklen wieder auf 0 setzen und die durchschnittliche Amplitude des Atmungszyklus aus der vorbestimmten Anzahl (Y4) von Atmungszyklen zu berechnen.

7. Gerät gemäß Anspruch 6, **dadurch gekennzeichnet, dass**
der zweite Faktor (F2) deutlich gleich 5/8 ist,
der dritte Hyperventilationsfaktor (F3) zwischen 100 % und 200 % inbegriffen ist und zum Beispiel deutlich gleich 140 % ist,
die siebten, achten, neunten und zehnten vorbestimmten Anzahlen (Y7; Y8; Y9; Y10) jeweils deutlich gleich 3; 2; 2; und 2 sind.

8. Gerät gemäß Anspruch 1 bis 7, **dadurch gekennzeichnet, dass**:
- wenn der gemessene Druck (P) unter einem vorbestimmten hohen Druckwert (PH) liegt, ein Zeitzähler hohen Drucks (TPH) wieder auf 0 gesetzt wird;
- wenn der Wert des Zeitzählers hohen Drucks (TPH) höher als eine maximale Zeit hohen Drucks (TMPH) ist und
* wenn der eingestellte maximale Druckwert (Pmaxi) unter einem vorbestimmten Wert eines Sicherheitsdrucks (PSEC) liegt, den Druck (P) dieses eingestellten maximalen Druckwertes (Pmaxi) steuern;
* wenn der der eingestellte minimale Druckwert (Pmini) höher als ein vorbestimmter Wert eines Sicherheitsdrucks (PSEC) ist, den Druck (P) dieses eingestellten minimalen Wertes eines Sicherheitsdrucks (Pmin) steuern;
* wenn die beiden vorherigen Bedingungen nicht erfüllt sind, den Druck (P) auf den Wert des Sicherheitsdrucks (PSEC) steuern;
anschließend
* den Zeitzähler hohen Drucks (TPH) wieder auf 0 setzen.

9. Gerät gemäß Anspruch 8, **dadurch gekennzeichnet, dass** der Wert hohen Drucks (PH) zwischen 10 mbar und 25 mbar inbegriffen ist und z. B. deutlich gleich 17 mbar ist,
die maximale Zeit hohen Drucks (TMPH) zwischen 1 und 100 Minuten inbegriffen ist und z. B. deutlich gleich 10 Minuten oder 30 Minuten ist,
der Wert des Sicherheitsdrucks (PSEC) deutlich gleich 8 mbar ist.

10. Gerät gemäß Anspruch 1 bis 9, **dadurch gekennzeichnet, dass** die Software angeordnet ist, um einen dem durchschnittlichen Luftdurchsatz während der Atmung des Patienten deutlich gleichen Luftaustritt zu messen,
- wenn der gemessene Luftaustritt höher ist als ein vorbestimmtes Austrittsniveau (NFM), die Steuerungen zur Druckerhöhung rückgängig machen.

11. Gerät gemäß Anspruch 10, **dadurch gekennzeichnet, dass** das vorbestimmte Austrittsniveau (NFM) deutlich gleich einem Austrittskoeffizienten (A) ist, multipliziert mit einem in der Maske gefilterten Luftdruck, der einem zusätzlichen Austrittskoeffizienten hinzugefügt wird, wobei der Austrittskoeffizient (A) zwischen 0 und 10 l/Minute mbar inbegriffen ist und z. B. deutlich gleich 2,5 l/Minute mbar ist und
der zusätzliche Austrittskoeffizient (B) zwischen 0 und 100 1/Minute inbegriffen ist und z. B. deutlich gleich 50 1/Min. ist.

12. Gerät gemäß Anspruch 1 bis 11, **dadurch gekennzeichnet, dass** die Software angeordnet ist, um:
- festzustellen, ob die gemessene Druckkurve Schwankungen aufweist, wie z. B. in einem Frequenzbereich (P1) inbegriffene akustische Vibrationen,
- die Zeit (RF1) des Vorhandenseins von Schwankungen zwischen zwei aufeinander folgenden festgestellten fehlenden Schwankungen und der Zeit (RFO) zwischen zwei aufeinander folgenden festgestellten vorhandenen Schwankungen fehlenden Schwankungen zu messen;
- wenn die Summe der gemessenen Zeiten des Fehlens und des Vorhandenseins von festgestellten Schwankungen (RFO; RF1) in einem vorübergehenden vorgeschriebenen Bereich (BIP; BSP) inbegriffen ist;
- wenn die gemessene Zeit vorhandener Schwankungen (RF1) größer als oder gleich einer Mindest-Schwankungszeit (TMRH) ist und
- wenn der Wert eines Zählers (CTAR) der seit dem vorletzten Mal, als die vorherigen zeitlichen Bedingungen erfüllt waren, abgelaufenen Zeit größer ist als eine vorgeschriebene Wartezeit (TAR), ein Achtel der vorbestimmten Druckerhöhung steuern (C8) und den Zähler der abgelaufenen Zeit (CTAR) wieder auf Null zu setzen.

13. Gerät gemäß Anspruch 12, **dadurch gekennzeichnet, dass** die Software angeordnet ist, um
- wenn die Summe der gemessenen Zeiten festgestellter fehlender und vorhandener Schwankungen (RF0; RF1) außerhalb des vorgeschriebenen zeitlichen Bereichs (BIP; BSP) liegt oder wenn die gemessene Zeit festgestellter vorhandener Schwankungen (RF1) unter einer Mindestzeit der Schwankungen (TMRH) liegt,
* die gemessene Zeit (RF0) festgestellter fehlender Schwankungen durch die Summe der Zeit der gemessenen fehlenden und vorhandenen Schwankungen (RF0; RF1) ersetzen und anschließend
* die gemessene Zeit der festgestellten vorhandenen Schwankungen (RF1) wieder auf 0 setzen; und
- wenn die Summe der gemessenen Zeit der festgestellten vorhandenen und fehlenden Schwankungen (RF0; RF1) den vorbestimmten zeitlichen Bereich (BIP; BSP) oder eine vorbestimmte maximale Zeit (TCMax) überschreitet, jede der gemessenen Zeiten der festgestellten vorhandenen und fehlenden Schwankungen (RF9; RF1) wieder auf Null setzen; und andernfalls
- jede der gemessenen Zeiten der festgestellten fehlenden und vorhandenen Schwankungen (RF0; RF1) wieder auf 0 setzen.

14. Gerät gemäß Anspruch 12 und 13, **dadurch gekennzeichnet, dass**
die maximale vorbestimmte Zeit deutlich gleich dem Zweifachen der Zeit (TCM) des durchschnittlichen Atmungszyklus der letzten drei gemessenen Zyklen ist:
- der vorgeschriebene zeitliche Bereich (BIP; BSP) deutlich zwischen 10 % und 120 % der berechneten durchschnittlichen Zykluszeit (TCM) inbegriffen ist;
- die Mindest-Schwankungszeit (TMRH) deutlich gleich 7 % der berechneten durchschnittlichen Zykluszeit (TCM) ist;
- die vorgeschriebene Wartezeit (TAR) zwischen 1 und 30 Minuten inbegriffen ist und z. B. deutlich gleich 1 Minute ist;
- die achte Steuerung (C8) der Druckerhöhung zwischen 0,1 mbar und 10 mbar inbegriffen ist und z. B. deutlich gleich 1 mbar ist;
- der Frequenzbereich (P1) der Schwankungsfeststellung deutlich zwischen 30 und 300 Hz inbegriffen ist.

15. Gerät gemäß Anspruch 1 bis 14, **dadurch gekennzeichnet, dass** die Software angeordnet ist, um die Chronologie der festgestellten Ereignisse zu speichern und z. B. nach einer Nacht die gespeicherte Chronologie abzulesen.

16. Gerät gemäß Anspruch 1 bis 15, **dadurch gekennzeichnet, dass** es einen Speicher der Chronologie der festgestellten Ereignisse umfasst, dessen Inhalt zum Ablesen geeignet ist, z. B. nach einer Nacht.
